(19) Europäisches Patentamt
European Patent Office
Office européen des brevets

(11) Veröffentlichungsnummer: **0 049 854**
**B1**

(12) # EUROPÄISCHE PATENTSCHRIFT

(45) Veröffentlichungstag der Patentschrift: **16.01.85**

(51) Int. Cl.[4]: **C 07 D 241/12,** C 07 D 213/53, A 01 N 43/40, A 01 N 43/60

(21) Anmeldenummer: **81107990.4**

(22) Anmeldetag: **06.10.81**

(54) **Pyridin- und Pyrazin-Derivate, Herstellung dieser Verbindungen, fungizide Mittel, die diese Verbindungen als Wirkstoffe enthalten sowie Verwendung solcher Verbindungen bzw. Mittel zur Bekämpfung von Fungi in der Landwirtschaft und im Gartenbau.**

(30) Priorität: **10.10.80 CH 7584/80**
**15.07.81 CH 4642/81**

(43) Veröffentlichungstag der Anmeldung: **21.04.82 Patentblatt 82/16**

(45) Bekanntmachung des Hinweises auf die Patenterteilung: **16.01.85 Patentblatt 85/3**

(84) Benannte Vertragsstaaten: **AT BE CH DE FR IT LI NL**

(56) Entgegenhaltungen: **EP - A - 0 007 679**

(73) Patentinhaber: **F. HOFFMANN-LA ROCHE & CO. Aktiengesellschaft, CH-4002 Basel (CH)**

(72) Erfinder: **Dorn, Franz, Dr., Bohnackerstrasse 5, CH-8157 Dielsdorf (CH)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

EP 0 049 854 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft Verbindungen der allgemeinen Formel

$$\begin{array}{l} \qquad R^3 \\ \qquad | \\ R^1-C-CH-R^2 \\ \quad\;\; \| \\ \quad\;\; N \\ \quad\;\; | \\ \quad\;\; OR^4 \end{array} \qquad \mathrm{I}$$

worin

$R^1$ 2-Halo-, 4-Halo- oder 2,4-Dihalo-phenyl,

$R^2$ 3-Pyridyl oder 2-Pyrazinyl,

$R^3$ Wasserstoff oder geradkettiges $C_{1-4}$-Alkyl und

$R^4$ $C_{1-6}$-Alkyl, $C_{3-6}$-Cycloalkyl, $C_{3-6}$-Alkenyl oder $C_{3-6}$-Alkinyl bedeuten,

und Säureadditionssalze dieser Verbindungen.

Die Verbindungen der Formel I und deren Säureadditionssalze besitzen fungizide Eigenschaften und eignen sich als fungizide Wirkstoffe, insbesondere zur Verwendung in der Landwirtschaft und im Gartenbau.

Die Erfindung betrifft ferner ein Verfahren zur Herstellung der Verbindungen der Formel I und ihrer Säureadditionssalze, Verbindungen der Formel I und Säureadditionssalze davon als fungizide Wirkstoffe, fungizide Mittel, die Verbindungen der Formel I oder Säureadditionssalze davon als Wirkstoffe enthalten, sowie die Verwendung solcher Verbindungen, Säureadditionssalze und Mittel zur Bekämpfung von Pilzen in der Landwirtschaft und im Gartenbau.

Auch die EP-A-7 679 beschreibt generisch 2-(3-Pyridyl)-acetophenon-O-alkyl-, alkenyl- und alkinyl-oxime, die am Benzolkern Halogensubstituenten tragen können, jedoch werden derartige Verbindungen dort nicht spezifisch offenbart. Darüber hinaus werden in dieser Patentpublikation nur pharmazeutische Eigenschaften der betreffenden Verbindungen erwähnt.

Der Ausdruck «Halo» in der Definition der Formel I umfasst Fluor, Chlor, Brom und Jod, wobei Chlor bevorzugt ist.

Die Halogenatome im Rest $R^1$ in der Bedeutung 2,4-Dihalo-phenyl können gleich oder verschieden sein.

Die Ausdrücke «$C_{1-6}$-Alkyl», «$C_{3-6}$-Alkenyl» und «$C_{3-6}$-Alkinyl» umfassen sowohl geradkettige als auch verzweigte Kohlenwasserstoffreste. Unter Alkyl sind je nach Anzahl der Kohlenstoffatome z.B. folgende Gruppen zu verstehen: Methyl, Äthyl, n-Propyl, Isopropyl, n-Butyl, tert.-Butyl, Isoamyl und n-Hexyl.

$R^4$ in der Bedeutung $C_{1-6}$-Alkyl, $C_{3-6}$-Alkenyl bzw. $C_{3-6}$-Alkinyl ist vorzugsweise $C_{1-4}$-Alkyl, $C_{3\ \mathrm{oder}\ 4}$-Alkenyl bzw. $C_{3\ \mathrm{oder}\ 4}$-Alkinyl. Eine besonders bevorzugte Alkenyl- bzw. Alkinylgruppe ist die Allyl- bzw. Propargylgruppe.

$R^1$ bedeutet vorzugsweise 4-Chlorphenyl oder 2,4-Dichlorphenyl, insbesondere die letztere Gruppe.

$R^3$ bedeutet vorzugsweise Wasserstoff, Methyl oder Äthyl, insbesondere Wasserstoff.

Eine besonders bevorzugte Verbindung der Formel I ist das 2',4'-Dichlor-2-(3-pyridyl)-acetophenon-O-methyloxim.

Weitere Vertreter von Verbindungen der Formel I sind:

4'-Chlor-2-(3-pyridyl)-acetophenon-O-allyloxim und 2',4'-Dichlor-2-(2-pyrazinyl)-acetophenon-O-isopropyloxim.

Durch das Vorliegen der C=N-Doppelbindung in den Verbindungen der Formel I tritt geometrische Isomerie auf. Zusätzlich können asymmetrische Kohlenstoffatome vorhanden sein, so dass die Verbindungen als optische Antipoden vorliegen können. Die Formel I soll demnach all diese möglichen isomeren Formen umfassen.

Als Säureadditionssalze der Verbindungen der Formel I kommen physiologisch verträgliche Salze in Frage. Hierzu gehören vorzugsweise Salze der Verbindungen I mit anorganischen oder organischen Säuren wie Salzsäure, Salpetersäure, Phosphorsäure, mono- und bifunktionellen Carbonsäuren und Hydroxycarbonsäuren, z.B. Essigsäure, Maleinsäure, Bernsteinsäure, Fumarsäure, Weinsäure, Zitronensäure, Salicylsäure, Sorbinsäure und Milchsäure, und Sulfonsäuren, z.B. 1,5-Naphthalin-disulfonsäure.

Das erfindungsgemässe Verfahren zur Herstellung der Verbindungen der Formel I und von deren Salzen ist dadurch gekennzeichnet, dass man

a) ein Oxim der Formel

$$\begin{array}{l} \qquad R^3 \\ \qquad | \\ R^1-C-CH-R^2 \\ \quad\;\; \| \\ \quad\;\; N \\ \quad\;\; | \\ \quad\;\; OH \end{array} \qquad \mathrm{II}$$

worin $R^1$, $R^2$ und $R^3$ die oben angegebenen Bedeutungen besitzen, mit einer Verbindung der Formel

$$R^4U \qquad \mathrm{III}$$

worin $R^4$ die oben angegebene Bedeutung besitzt und

U eine Abgangsgruppe, z.B. Chlor, Brom, Jod, Mesyloxy, Tosyloxy oder einen Alkylsulfatrest, bedeutet, umsetzt, oder

b) ein Keton der Formel

$$\begin{array}{l} \qquad R^3 \\ \qquad | \\ R^1-C-CH-R^2 \\ \quad\;\; \| \\ \quad\;\; O \end{array} \qquad \mathrm{IV}$$

worin $R^1$, $R^2$ und $R^3$ die oben angegebenen Bedeutungen besitzen, mit einem O-substituierten Hydroxylamin der Formel

$$R^{41}ONH_2 \qquad \mathrm{V}$$

worin $R^{41}$ $C_{1-6}$-Alkyl, $C_{3-6}$-Cycloalkyl oder $C_{3-6}$-Alkenyl bedeutet, umsetzt, und erwünschtenfalls

eine erhaltene Verbindung der Formel I in ein Säureadditionssalz überführt.

Die Verfahrensvariante a) kann durchgeführt werden, indem man das Ausgangsmaterial der Formel II mit der Verbindung der Formel III, zweckmässigerweise in Gegenwart einer Base, in einem organischen Lösungsmittel und in einem Temperaturbereich zwischen 0 °C und der Rückflusstemperatur des Reaktionsgemisches versetzt. Das Lösungsmittel kann protisch oder nicht protisch sein. Im Falle von protischen Lösungsmitteln, wie z.B. Alkoholen, insbesondere Methanol oder Äthanol, ist die verwendete Base vorzugsweise ein Alkalimetallhydroxid, z.B. Natrium- oder Kaliumhydroxid, oder ein Alkalimetallalkoholat. Bei Verwendung von nichtprotischen Lösungsmitteln, wie z.B. Äthern oder ätherartigen Verbindungen, insbesondere Tetrahydrofuran oder Dimethoxyäthan, und Dialkylamiden, insbesondere Dimethylformamid, ist die Base vorzugsweise ein Alkalimetallhydrid, z.B. Natriumhydrid.

In einer bevorzugten Ausführungsform dieser Variante werden Natriumhydrid als Base und ein Äther oder eine ätherartige Verbindung, insbesondere Tetrahydrofuran oder Dimethoxyäthan, oder ein Dialkylamid, insbesondere Dimethylformamid, als Lösungsmittel verwendet.

Gemäss Verfahrensvariante b), die zu Verbindungen der Formel I führt, worin $R^4$ $C_{1-6}$-Alkyl, $C_{3-6}$-Cycloalkyl oder $C_{3-6}$-Alkenyl bedeutet, erfolgt die Umsetzung zweckmässigerweise in einem organischen Lösungsmittel, z.B. einem Alkohol, wie Methanol oder Äthanol, einem Dialkylamid, wie Dimethylformamid, oder einem tertiären Amin, wie Pyridin. Man arbeitet vorzugsweise in einem Temperaturbereich zwischen der Raumtemperatur und der Rückflusstemperatur des Reaktionsgemisches. Da das Ausgangsmaterial der Formel V vorzugsweise in Form eines Säureadditionssalzes, z.B. des Hydrochlorids oder Hydrosulfats, eingesetzt wird, wird dem Reaktionsgemisch zweckmässigerweise eine Base, wie Natrium- oder Kaliumcarbonat, Triäthylamin oder Pyridin, zugesetzt.

Zur Herstellung von Säureadditionssalzen können erwünschtenfalls die Verbindungen der Formel I mit anorganischen oder organischen Säuren, wie z.B. Salzsäure, Salpetersäure, Phosphorsäure, mono- und bifunktionellen Carbonsäuren und Hydroxycarbonsäuren oder Sulfonsäuren, umgesetzt werden.

Die Isolierung und Reinigung der so hergestellten Verbindungen der Formel I und deren Säureadditionssalze erfolgen nach an sich bekannten Methoden.

Die als Ausgangsmaterialien verwendbaren Verbindungen der Formeln II, III, IV und V sind entweder bekannt oder können nach an sich bekannten Methoden hergestellt werden. Beispielsweise können die Oxime der Formel II aus den entsprechenden Ketonen der Formel IV durch Umsetzung mit Hydroxylamin, und die Ketone selbst gemäss den in der DOS 2 221 546, der DOS 2 800 010 und der britischen Patentschrift Nr. 2 015 524 beschriebenen Verfahren hergestellt werden.

Die erfindungsgemässen Verbindungen, d.h. die Verbindungen der Formel I und deren Säureadditionssalze, besitzen fungizide Wirkung und können dementsprechend zur Bekämpfung von Fungi in der Landwirtschaft und im Gartenbau Verwendung finden. Sie eignen sich insbesondere zur Vernichtung oder Eindämmung von phytopathogenen Pilzen auf Pflanzenteilen, z.B. Blättern, Stengeln, Wurzeln, Knollen, Früchten oder Blüten, und auf Saatgut sowie im Erdboden und sind besonders wirksam bei der Bekämpfung von echten Mehltaupilzen, wie beispielsweise Erysiphe graminis (Getreidemehltau), Erysiphe cichoracearum (Gurkenmehltau), Podosphaera leucotricha (Apfelmehltau) und Sphaerotheca pannosa (Rosenmehltau); von Venturia inaequalis (Apfelschorf); und von Rostpilzen, wie beispielsweise solchen der Gattungen Puccinia, Uromyces und Hemileia, insbesondere Puccinia coronata (Haferkronenrost), Puccinia recondita (Getreidebraunrost), Uromyces appendiculatus (Bohnenrost) und Hemileia vastatrix (Kaffeerost).

Ferner wirken verschiedene dieser Verbindungen auch gegen phytopathogene Pilze der folgenden Gattungen:

Helminthosporium, Rhizoctonia, Septoria, Cercospora, Corticium, Tilletia und Ustilago.

Einzelne Vertreter dieser Verbindungsklasse besitzen zudem eine ausgeprägte Wirkung gegen holzzerstörende Pilze, wie z.B. Caniophora puteana und Lenzites trabea. Ausserdem wirken einzelne der Verbindungen auch gegen phytopathogene Bakterien, wie z.B. Xanthomonas oryzae.

Die erfindungsgemässen Verbindungen zeichnen sich durch systemische Wirkung sowie durch eine gute Pflanzenverträglichkeit aus.

Die erfindungsgemässen Verbindungen wirken unter Gewächshausbedingungen bereits bei einer Konzentration von 5 mg bis 500 mg Wirksubstanz pro Liter Spritzbrühe. Im Freiland werden vorteilhaft Konzentrationen von 50 g bis 1000 g Wirksubstanz der Formel I pro Hektar und Behandlung zur Anwendung gebracht. Beispielsweise wird zur erfolgreichen Bekämpfung von Apfelmehltau und Apfelschorf eine Konzentration von 50 g bis 400 g Wirksubstanz der Formel I pro Hektar und Behandlung benützt. Zur Bekämpfung von samenbürtigen Pilzen im Beizverfahren werden mit Vorteil 0,1 g bis 2,5 g Wirksubstanz der Formel I pro kg Samen verwendet.

Die erfindungsgemässen Verbindungen können in verschiedenartigen Mitteln, z.B. Spritzbrühen, wässrigen Suspensionen, Emulsionen, emulgierbaren Konzentraten und pulverförmigen Präparaten, formuliert werden. Die erfindungsgemässen fungiziden Mittel sind dadurch gekennzeichnet, dass sie eine wirksame Menge von mindestens einer Verbindung der allgemeinen Formel I, wie oben definiert, oder einem Säureadditionssalz einer solchen Verbindung sowie inertes Trägermaterial enthalten.

Im allgemeinen enthält ein Mittel, je nach dessen Art, zwischen 0,0001 und 95 Gewichtsprozent an erfindungsgemässer Verbindung bzw. erfindungsgemässen Verbindungen als Wirkstoff(en).

Zur Herstellung von pulverförmigen Präparaten kommen verschiedene inerte pulverförmige Trägerstoffe in Frage, wie z.B. Kaolin, Bentonit, Talkum, Schlämmkreide, Magnesiumcarbonat und Kieselgur. Die aktiven Komponenten werden mit solchen Trägerstoffen vermischt, z.B. durch Zusammenmahlen; oder man imprägniert den inerten Trägerstoff mit einer Lösung der aktiven Komponenten und entfernt dann das Lösungsmittel durch Abdunsten, Erhitzen oder durch Absaugen unter vermindertem Druck. Solche pulverförmigen Präparate können als Stäubemittel mit Hilfe der üblichen Verstäubergeräte auf die zu schützenden Pflanzen aufgebracht werden. Durch Zusatz von Netz- und/oder Dispergiermitteln kann man solche pulverförmigen Präparate mit Wasser leicht benetzbar machen, so dass sie in Form von wässrigen Suspensionen als Spritzmittel anwendbar sind.

Zur Herstellung emulgierbarer Konzentrate können die aktiven Stoffe beispielsweise mit einem Emulgiermittel gemischt oder auch in einem inerten Lösungsmittel gelöst und mit einem Emulgator gemischt werden. Durch Verdünnen solcher Konzentrate mit Wasser erhält man gebrauchsfertige Emulsionen. Derartige Konzentrate können 5 bis 95 Gewichtsprozent, insbesondere 25 bis 75 Gewichtsprozent, Wirkstoff enthalten.

Die erfindungsgemässen fungiziden Mittel können neben den Wirkstoffen der Formel I auch andere Wirkstoffe enthalten, z.B. anderweitige fungizide Mittel, insektizide und akarizide Mittel, anderweitige Bakterizide, Pflanzenwachstumsregulatoren und Düngemittel. Solche Kombinationsmittel eignen sich zur Verbreiterung des Wirkungsspektrums oder zur spezifischen Beeinflussung des Pflanzenwachstums.

Die Verwendung der erfindungsgemässen Mittel kann nach den im Pflanzenschutz bzw. in der Landwirtschaft üblichen Applikationsmethoden erfolgen. Das erfindungsgemässe Verfahren zur Bekämpfung von Fungi ist dadurch gekennzeichnet, dass man das zu schützende Gut, z.B. Pflanzen, Pflanzenteile bzw. Samen, mit einer wirksamen Menge einer erfindungsgemässen Verbindung bzw. eines erfindungsgemässen Mittels behandelt.

Die nachstehenden Beispiele illustrieren die Erfindung. Alle Temperaturen sind in °C angegeben.

I. Herstellung der Wirkstoffe

Beispiel 1

Eine Lösung von 13,3 g 2',4'-Dichlor-2-(3-pyridyl)-acetophenon in 40 ml Äthanol wird mit 10 g Natriumcarbonat und 8,3 g O-Methylhydroxylamin-hydrochlorid versetzt und anschliessend unter Rühren auf Rückflusstemperatur erhitzt. Nach 4 Stunden wird das Reaktionsgemisch auf Eis gegossen und mit Essigester extrahiert. Die organische Phase wird gewaschen, über Natriumsulfat getrocknet und unter vermindertem Druck eingeengt. Man erhält 2',4'Dichlor-2-(3-pyridyl)-acetophenon-O-methyloxim als E,Z-Isomerengemisch in Form eines gelblichen Öls. Bei der chromatographischen Trennung an Silicagel mit n-Hexan/Essigester (4:1) wird zuerst das E-Isomere, $n_D^{20}$ = 1,5845, dann das Z-Isomere, $n_D^{20}$ = 1,5745, eluiert.

Auf analoge Weise erhält man:

Aus 4-Chlor -2- (3-pyridyl)-acetophenon und O-Methyl-hydroxylamin - hydrochlorid das 4'-Chlor -2- (3-pyridyl)-acetophenon-O-methyloxim, Smp. 55–58°.

Aus 4'-Chlor-2- (2-pyrazinyl)-acetophenon und O-Methylhydroxylamin-hydrochlorid das 4'-Chlor-2- (2-pyrazinyl)-acetophenon-O- methyloxim, Smp. 53–58°.

Aus 4'-Chlor-2- (2-pyrazinyl) -acetophenon und O-Äthylhydroxylamin-hydrochlorid das 4'-Chlor-2- (2-pyrazinyl)-acetophenon-O-äthyloxim, Smp. 44–46°.

Aus 4'-Chlor-2- (2-pyrazinyl)-acetophenon und O-Allylhydroxylamin-hydrochlorid das 4'-Chlor-2- (2-pyrazinyl)-acetophenon-O-allyloxim als gelbliches Öl.

Aus 2',4'-Dichlor-2- (3-pyridyl)-acetophenon und O-Allylhydroxylamin-hydrochlorid das 2',4'-Dichlor-2- (3-pyridyl)-acetophenon-O- allyloxim als farbloses Öl.

Beispiel 2

3 g 2'-Chlor-2-(2-pyrazinyl)-acetophenon und 0,8 g O-Methylhydroxylamin-hydrochlorid werden in 10 ml Pyridin gelöst und während 30 Minuten auf 80° erhitzt. Dann wird das Pyridin unter vermindertem Druck entfernt und Wasser zum Rohprodukt zugefügt. Hierauf extrahiert man mit Essigester, trocknet die organische Phase über Natriumsulfat und engt ein. Man erhält 2'-Chlor-2-(2-pyrazinyl) -acetophenon-O- methyloxim als Öl.

Auf analoge Weise erhält man:

Aus 4'-Fluor-2- (2-pyrazinyl)-acetophenon und O-Methylhydroxylamin-hydrochlorid das 4'-Fluor-2- (2-pyrazinyl)-acetophenon-O-methyloxim, Smp. 52–54°.

Aus 4'-Brom-2- (2-pyrazinyl)-acetophenon und O-Methylhydroxylamin-hydrochlorid das 4'-Brom-2- (2-pyrazinyl)-acetophenon-O-methyloxim, Smp. 81–84°.

Aus 4'-Chlor-2- (2-pyrazinyl)-propiophenon und O-Methylhydroxylamin-hydrochlorid das 4'-Chlor-2- (2-pyrazinyl)-propiophenon-O-methyloxim als Öl.

Aus 2',4'-Dichlor-2- (2-pyrazinyl)- acetophenon und O-Methylhydroxylamin-hydrochlorid das 2',4'-Dichlor- 2-(2-pyrazinyl)-acetophenon-O-methyloxim als Öl.

Aus 2',4'-Dichlor-2- (2-pyrazinyl)- acetophenon und O-Äthylhydroxylamin-hydrochlorid das 2',4'-Dichlor-2- (2-pyrazinyl)-acetophenon-O-äthyloxim als Öl.

Aus 2',4'-Dichlor-2- (2-pyrazinyl)- acetophenon und O-Allylhydroxylamin-hydrochlorid das

2',4'-Dichlor-2- (2-pyrazinyl)-acetophenon-O-allyloxim als Öl.

Aus 2',4'-Dichlor-2- (2-pyrazinyl)-propiophenon und O-Methylhydroxylamin-hydrochlorid das 2',4'Dichlor-2- (2-pyrazinyl)-propiophenon-O- methyloxim als Öl.

Beispiel 3

Eine Lösung von 3 g 2',4'-Dichlor-2- (3-pyridyl)-acetophenonoxim in 25 ml Dimethoxyäthan wird portionenweise mit 0,51 g Natriumhydrid-Dispersion (55% in Öl) versetzt und 30 Minuten bei Raumtemperatur gerührt. Darauf gibt man 2,0 g Äthyljodid zu und erhitzt das Reaktionsgemisch auf Rückflusstemperatur. Nach 4 Stunden wird auf Eis gegossen, mit Essigester extrahiert, die organische Phase über Natriumsulfat getrocknet und unter vermindertem Druck eingeengt. Das Rohprodukt wird durch Chromatographie an Silicagel gereinigt. Man erhält 2',4'-Dichlor-2- (3-pyridyl) -acetophenon-O-äthyloxim als gelbliches Öl.

Auf analoge Weise erhält man:

Aus 2',4'-Dichlor-2- (3-pyridyl)-acetophenonoxim und Isopropylbromid das 2',4'-Dichlor-2- (3-pyridyl)- acetophenon-O-isopropyloxim als gelbliches Öl.

Aus 2',4'-Dichlor-2- (3-pyridyl)-acetophenonoxim und Propargylbromid das 2',4'-Dichlor-2- (3-pyridyl)- acetophenon-O-propargyloxim, Smp. 60–62°.

Aus 4'-Chlor-2-(2-pyrazinyl)-acetophenonoxim und Propargylbromid das 4'-Chlor-2- (2-pyrazinyl)- acetophenon-O-propargyloxim, Smp. 76–77°.

Aus 2',4'-Dichlor-2- (2-pyrazinyl)-acetophenonoxim und Propargylbromid das 2',4'-Dichlor-2- (2-pyrazinyl)- acetophenon-O- propargyloxim als Öl.

II. Herstellung der Ausgangsmaterialien

Beispiel 4

Das als Ausgangsmaterial im Beispiel 1 benötigte 2',4'-Dichlor-2- (3-pyridyl)-acetophenon kann wie folgt hergestellt werden:

Ein Gemisch von 27,6 g 2,4-Dichlorbenzoesäure-äthylester und 20,81 g 3-Pyridylessigsäureäthylester bei 20–25° wird portionenweise mit 10,59 g Natriummethylat versetzt. Das Reaktionsgemisch wird anschliessend auf 65–70° erhitzt, und entstehende leichtflüchtige Produkte werden mit trockenem Stickstoff abgeblasen. Nach 20 Stunden wird mit 40 ml konzentrierter Salzsäure versetzt und 18 Stunden bei Rückflusstemperatur erhitzt. Das Gemisch wird mit Diäthyläther gewaschen und die wässerige Phase durch Zugabe von konzentriertem Ammoniak basisch gestellt und mit Methylenchlorid extrahiert. Die organische Phase wird eingeengt und das Rohprodukt an Kieselgel mit Methylenchlorid/Methanol (98:2) chromatographiert. Das Produkt, 2',4'-Dichlor-2- (3-pyridyl)-acetophenon, kann aus Diäthyläther/n-Pentan zur Kristallisation gebracht werden und schmilzt bei 55–56°.

Beispiel 5

Das als Ausgangsmaterial im Beispiel 2 benötigte 2',4'-Dichlor-2- (2-pyrazinyl)-acetophenon kann wie folgt hergestellt werden:

Zu einer Suspension von Natriumamid in flüssigem Ammoniak (aus 200 ml Ammoniak und 4,83 g Natrium) bei −30° tropft man 20 g 2-Methylpyrazin und lässt 30 Minuten rühren.

Dann wird 20,5 g 2,4-Dichlorbenzoesäuremethylester langsam zugetropft und 1 Stunde bei −30° reagieren gelassen. Nun wird mit Ammoniumchlorid versetzt, Ammoniak abgetrieben und Diäthyläther zugefügt. Die organische Phase wird mit Wasser gewaschen, über Natriumsulfat getrocknet und eingeengt. Mit wenig Äthanol versetzt, kristallisiert 2',4'-Dichlor-2- (2-pyrazinyl)-acetophenon aus dem Reaktionsrohprodukt aus. Das Produkt schmilzt bei 96–98°.

Auf analoge Weise erhält man:

Aus 2-Methylpyrazin und 2-Chlorbenzoesäuremethylester das 2'-Chlor-2- (2-pyrazinyl)-acetophenon als Öl.

Aus 2-Methylpyrazin und 4-Fluorbenzoesäuremethylester das 4'-Fluor-2-(2-pyrazinyl)-acetophenon, Smp. 100–103°.

Aus 2-Methylpyrazin und 4-Brombenzoesäuremethylester das 4'-Brom-2-(2-pyrazinyl)-acetophenon, Smp. 121–122°.

Aus 2-Äthylpyrazin und 4-Chlorbenzoesäuremethylester das 4'-Chlor-2-(2-pyrazinyl)-propiophenon, Smp. 85°.

Aus 2-Äthylpyrazin und 2,4-Dichlorbenzoesäuremethylester das 2',4'-Dichlor-2- (2-pyrazinyl)-propiophenon als Öl.

Beispiel 6

Das als Ausgangsmaterial im Beispiel 3 benötigte 4'-Chlor-2- (2-pyrazinyl)-acetophenonoxim kann wie folgt hergestellt werden:

10 g 4'-Chlor-2-(2-pyrazinyl)-acetophenon, 10 g Hydroxylamin-hydrochlorid und 12 g wasserfreies Soda werden in 100 ml Äthanol während 2 Stunden bei 60° gerührt. Dann versetzt man mit Wasser, extrahiert mit Essigsäure, trocknet die organische Phase über Natriumsulfat und engt anschliessend ein. Der Rest wird aus Aceton/n-Hexan kristallisiert. Man erhält 4'-Chlor-2- (2-pyrazinyl)- acetophenonoxim, Smp. 134°.

Auf analoge Weise erhält man:

Aus 2',4'-Dichlor-2-(2-pyrazinyl)-acetophenon und Hydroxylamin-hydrochlorid das 2',4'-Dichlor-2- (2-pyrazinyl)- acetophenonoxim als zähes Öl.

Aus 2',4'-Dichlor-2-(3-pyridyl)-acetophenon und Hydroxylamin-hydrochlorid das 2',4'-Dichlor-2- (3-pyridyl)-acetophenonoxim, Smp. 134–136°.

III. Formulierungsbeispiele

Beispiel 7

1. Spritzpulver (für flüssige oder unter 75° schmelzende Wirkstoffe)

| | Gewichtsteile |
|---|---|
| Wirkstoff der Formel I | 50 |
| Hydratisierte Kieselsäure | 37 |
| Kaolin | 5 |
| Alkylphenoläthoxylat | 4 |
| Natrium-polynaphthalinsulfonat | 4 |
| | 100 |

Der flüssige oder geschmolzene Wirkstoff wird auf die Kieselsäure aufgezogen, die übrigen Komponenten zugemischt und das Ganze in einer geeigneten Mühle feingemahlen.

2. Spritzpulver (für feste, über 75° schmelzende Wirkstoffe)

| | Gewichtsteile |
|---|---|
| Wirkstoff der Formel I | 50 |
| Hydratisierte Kieselsäure | 5 |
| Kaolin | 42 |
| Natrium-laurylsulfat | 1 |
| Natrium-lignosulfonat | 2 |
| | 100 |

Die Komponenten werden miteinander vermischt und das Ganze in einer geeigneten Mühle feingemahlen.

Beispiel 8

Emulgierbares Konzentrat (für bei 20–25° flüssige Wirkstoffe)

| | Gewichtsteile |
|---|---|
| Wirkstoff der Formel I | 500 |
| Ricinusöl-äthoxylat | 100 |
| Calcium-dodecylbenzolsulfonat | 25 |
| Gemisch von | |
| $C_{10}$-Alkylbenzolen | ad 1000 Vol.-Teile |

Die Komponenten werden miteinander vermischt, bis eine klare Lösung entsteht.

**Patentansprüche für die Vertragsstaaten: BE, CH, DE, FR, IT, LI, NL**

1. Verbindungen der allgemeinen Formel

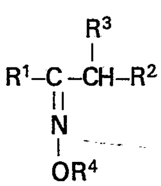
I

worin
$R^1$ 2-Halo-, 4-Halo- oder 2,4-Dihalo-phenyl,
$R^2$ 3-Pyridyl oder 2-Pyrazinyl,
$R^3$ Wasserstoff oder geradkettiges $C_{1-4}$-Alkyl und
$R^4$ $C_{1-6}$-Alkyl, $C_{3-6}$-Cycloalkyl, $C_{3-6}$-Alkenyl oder $C_{3-6}$-Alkinyl bedeuten, und Säureadditionssalze dieser Verbindungen.

2. Verbindungen nach Anspruch 1, worin $R^1$ 4-Chlorphenyl oder 2,4-Dichlorphenyl bedeutet.

3. Verbindungen nach Anspruch 1 oder 2, worin $R^3$ Wasserstoff bedeutet.

4. 2',4'-Dichlor-2- (3-pyridyl)-acetophenon-O-methyloxim.

5. Eine Verbindung nach Anspruch 1, der allgemeinen Formel

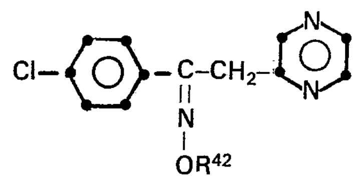

worin $R^{42}$ Methyl, Äthyl oder Allyl bedeutet.

6. Eine Verbindung nach Anspruch 1, der allgemeinen Formel

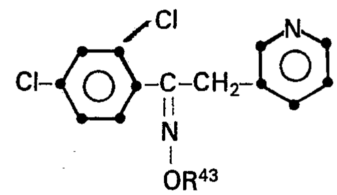

worin $R^{43}$ Äthyl, Isopropyl oder Propargyl bedeutet.

7. Eine Verbindung nach Anspruch 1, der allgemeinen Formel

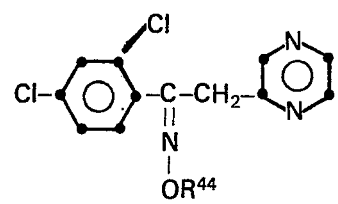

worin $R^{44}$ Methyl, Äthyl, Allyl oder Propargyl bedeutet.

8. Verbindungen nach einem der Ansprüche 1 bis 7 als fungizide Wirkstoffe.

9. Fungizides Mittel, dadurch gekennzeichnet, dass es eine wirksame Menge mindestens einer Verbindung der allgemeinen Formel I nach Anspruch 1 oder eines Säureadditionssalzes einer solchen Verbindung, sowie inertes Trägermaterial enthält.

10. Fungizides Mittel nach Anspruch 9, dadurch gekennzeichnet, dass es eine wirksame Menge 2',4'-Dichlor-2-(3-pyridyl)-acetophenon-O-methyloxim sowie inertes Trägermaterial enthält.

11. Verfahren zur Herstellung von Verbindungen der allgemeinen Formel I nach Anspruch 1 und von deren Säureadditionssalzen, dadurch gekennzeichnet, dass man

a) ein Oxim der Formel

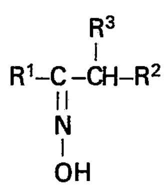
II

worin $R^1$, $R^2$ und $R^3$ die oben angegebenen Bedeutungen besitzen, mit einer Verbindung der Formel

$R^4U$ III

worin $R^4$ die oben angegebene Bedeutung besitzt und U eine Abgangsgruppe bedeutet, umsetzt, oder

b) ein Keton der Formel

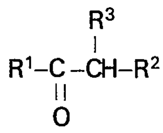

IV

worin $R^1$, $R^2$ und $R^3$ die oben angegebenen Bedeutungen besitzen, mit einem O-substituierten Hydroxylamin der Formel

$R^{41}ONH_2$ V

worin $R^{41}$ $C_{1-6}$-Alkyl, $C_{3-6}$-Cycloalkyl oder $C_{3-6}$-Alkenyl bedeutet, umsetzt, und erwünschtenfalls eine erhaltene Verbindung der Formel I in ein Säureadditionssalz überführt.

12. Verfahren zur Bekämpfung von Fungi in der Landwirtschaft und im Gartenbau, dadurch gekennzeichnet, dass man das zu schützende Gut mit einer wirksamen Menge mindestens einer der in den Ansprüchen 1 bis 7 genannten Verbindungen bzw. eines der in den Ansprüchen 9 und 10 genannten Mittel behandelt.

13. Verwendung einer der in den Ansprüchen 1 bis 7 genannten Verbindungen bzw. eines der in den Ansprüchen 9 und 10 genannten Mittel zur Bekämpfung von Fungi in der Landwirtschaft und im Gartenbau.

**Patentansprüche für den Vertragsstaat: AT**

1. Fungizides Mittel, dadurch gekennzeichnet, dass es eine wirksame Menge mindestens einer Verbindung der allgemeinen Formel

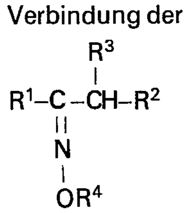

I

worin

$R^1$ 2-Halo-, 4-Halog- oder 2,4-Dihalo-phenyl,
$R^2$ 3-Pyridyl oder 2-Pyrazinyl,
$R^3$ Wasserstoff oder geradkettiges $C_{1-4}$-Alkyl und
$R^4$ $C_{1-6}$-Alkyl, $C_{3-6}$-Cycloalkyl, $C_{3-6}$-Alkenyl oder $C_{3-6}$-Alkinyl bedeuten, oder eines Säureadditionssalzes einer solchen Verbindung, sowie inertes Trägermaterial enthält.

2. Fungizides Mittel nach Anspruch 1, worin $R^1$ 4-Chlorphenyl oder 2,4-Dichlorphenyl bedeutet.

3. Fungizides Mittel nach Anspruch 1 oder 2, worin $R^3$ Wasserstoff bedeutet.

4. Fungizides Mittel nach Anspruch 1, dadurch gekennzeichnet, dass es eine wirksame Menge 2',4'-Dichlor-2-(3-pyridyl)-acetophenon-O-methyloxim sowie inertes Trägermaterial enthält.

5. Fungizides Mittel nach Anspruch 1, dadurch gekennzeichnet, dass es eine wirksame Menge einer Verbindung der allgemeinen Formel

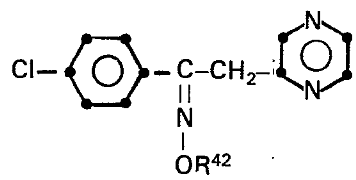

worin $R^{42}$ Methyl, Äthyl oder Allyl bedeutet, sowie inertes Trägermaterial enthält.

6. Fungizides Mittel nach Anspruch 1, dadurch gekennzeichnet, dass es eine wirksame Menge einer Verbindung der allgemeinen Formel

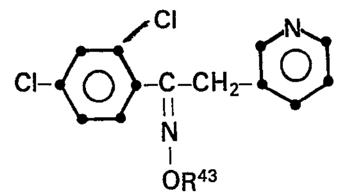

worin $R^{43}$ Äthyl, Isopropyl oder Propargyl bedeutet, sowie inertes Trägermaterial enthält.

7. Fungizides Mittel nach Anspruch 1, dadurch gekennzeichnet, dass es eine wirksame Menge einer Verbindung der allgemeinen Formel

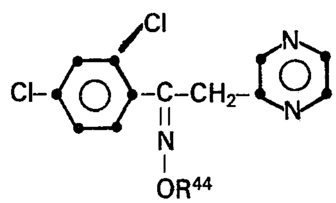

worin $R^{44}$ Methyl, Äthyl, Allyl oder Propargyl bedeutet, sowie inertes Trägermaterial enthält.

8. Verfahren zur Herstellung von Verbindungen der allgemeinen Formel

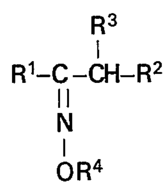

I

worin

$R^1$ 2-Halo-, 4-Halo- oder 2,4-Dichalo-phenyl,
$R^2$ 3-Pyridyl oder 2-Pyrazinyl,
$R^3$ Wasserstoff oder geradkettiges $C_{1-4}$-Alkyl und
$R^4$ $C_{1-6}$-Alkyl, $C_{3-6}$-Cycloalkyl, $C_{3-6}$-Alkenyl oder $C_{3-6}$-Alkinyl bedeuten, und von deren Säureadditionssalzen, dadurch gekennzeichnet, dass man

a) ein Oxim der Formel

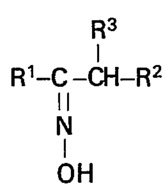

II

worin $R^1$, $R^2$ und $R^3$ die oben angegebenen Be-

deutungen besitzen, mit einer Verbindung der Formel

$R^4U$ III

worin $R^4$ die oben angegebene Bedeutung besitzt und U eine Abgangsgruppe bedeutet, umsetzt, oder

b) ein Keton der Formel

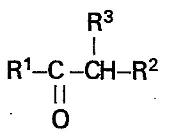

IV

worin $R^1$, $R^2$ und $R^3$ die oben angegebenen Bedeutungen besitzen, mit einem O-substituierten Hydroxylamin der Formel

$R^{41}ONH_2$ V

worin $R^{41}$ $C_{1-6}$-Alkyl, $C_{3-6}$-Cycloalkyl oder $C_{3-6}$-Alkenyl, bedeutet, umsetzt, und erwünschtenfalls eine erhaltene Verbindung der Formel I in ein Säureadditionssalz überführt.

9. Verfahren zur Herstellung eines fungiziden Mittels, dadurch gekennzeichnet, dass man mindestens eine der in den Ansprüchen 1 bis 6 genannten Verbindungen mit inertem Trägermaterial vermischt.

10. Verfahren zur Herstellung eines fungiziden Mittels, dadurch gekennzeichnet, dass man mindestens eine der in Anspruch 7 genannten Verbindungen mit inertem Trägermaterial vermischt.

11. Verfahren zur Bekämpfung von Fungi in der Landwirtschaft und im Gartenbau, dadurch gekennzeichnet, dass man das zu schützende Gut mit einer wirksamen Menge mindestens einer der in den Ansprüchen 1 bis 6 genannten Verbindungen behandelt.

12. Verfahren zur Bekämpfung von Fungi in der Landwirtschaft und im Gartenbau, dadurch gekennzeichnet, dass man das zu schützende Gut mir einer wirksamenMenge einer der in Anspruch 7 genannten Verbindungen behandelt.

13. Verwendung einer bzw. eines der in den Ansprüchen 1 bis 6 genannten Verbindungen bzw. Mittel zur Bekämpfung von Fungi in der Landwirtschaft und im Gartenbau.

14. Verwendung einer bzw. eines der in Anspruch 7 genannten Verbindung bzw. Mittel zur Bekämpfung von Fungi in der Landwirtschaft und im Gartenbau.

**Revendications pour les Etats contractants: BE, CH, DE, FR, IT, LI, NL**

1. Composés de formule générale

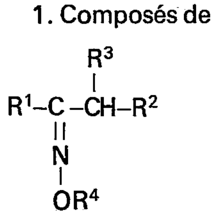

I

où

$R^1$ représente un 2-halo-, 4-halo-, ou 2,4-dihalo-phényle,

$R^2$ représente un 3-pyridyle ou un 2-pyrazinyle,

$R^3$ représente un hydrogène ou un alcoyle en $C_1$ à $C_4$ à chaîne droite et

$R^4$ représente un alcoyle en $C_1$ à $C_6$, un cycloalcoyle en $C_3$ à $C_6$, un alcényle en $C_3$ à $C_6$ ou un alcynyle en $C_3$ à $C_6$,

et les sels d'addition d'acides de ces composés.

2. Composés selon la revendication 1, où $R^1$ représente un 4-chlorophényle ou un 2,4-dichlorophényle.

3. Composés selon l'une des revendications 1 et 2, où $R^3$ représente un hydrogène.

4. 2',4'-Dichloro-2(3-pyridyl) -acétophénone-O-méthyloxime.

5. Composé selon la revendication 1, de formule générale

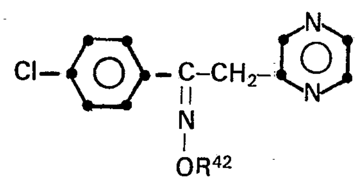

où $R^{42}$ représente un méthyle, un éthyle ou un allyle.

6. Composé selon la revendication 1, de formule générale

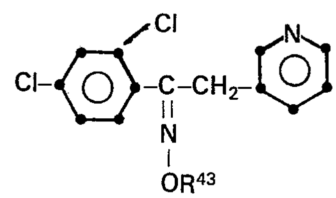

où $R^{43}$ représente un éthyle, un isopropyle ou un propargyle.

7. Composé selon la revendication 1, de formule générale

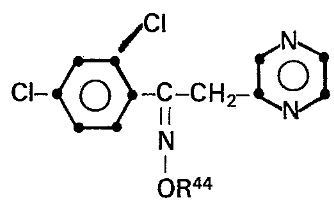

où $R^{44}$ représente un méthyle, un éthyle, un allyle ou un propargly.

8. Composés selon l'une des revendications 1 à 7 comme matières actives fongicides.

9. Agent fongicide caractérisé en ce qu'il contient une quantité efficace d'au moins un composé de formule générale I selon la revendication 1 ou d'un sel d'addition d'acide d'un tel composé, ainsi qu'un support inerte.

10. Agent fongicide selon la revendication 9, caractérisé en ce qu'il contient une quantité efficace de 2',4'-dichloro-2-(3-pyridyl)-acétophénone-O-méthyloxime, ainsi qu'un support inerte.

11. Procédé de préparation de composés de

formule générale I selon la revendication 1 et de leurs sels d'addition d'acides, caractérisé en ce que

a) on fait réagir un oxime de formule

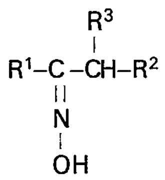

II

où $R^1$, $R^2$ et $R^3$ ont les significations données ci-dessus, avec un composé de formule

$R^4U$ III

où $R^4$ a la signification donnée ci-dessus, et U représente un groupe sortant, ou

b) on fait réagir une cétone de formule

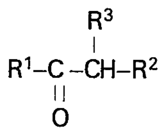

IV

où $R^1$, $R^2$ et $R^3$ ont les significations données ci-dessus, avec une hydroxylamine O-substituée de formule

$R^{41}ONH_2$ V

où $R^{41}$ représente un alcoyle en $C_1$ à $C_6$, un cycloalcoyle en $C_3$ à $C_6$ ou un alcényle en $C_3$ à $C_6$, et si on le désire on transforme un composé de formule I obtenu en un sel d'addition d'acide.

12. Procédé de lutte contre les champignons en agriculture et en horticulture, caractérisé en ce qu'on traite la matière à protéger avec une quantité efficace d'au moins un des composés mentionnés dans les revendications 1 à 7 ou selon les cas d'un des agents mentionnés dans les revendications 9 et 10.

13. Application d'un des composés mentionnés dans les revendications 1 à 7 ou selon les cas d'un des agents mentionnés dans les revendications 9 et 10 aux fins de lutte contre les champignons en agriculture et en horticulture.

**Revendications pour l'Etat contractant: AT**

1. Agent fongicide caractérisé en ce qu'il contient une quantité efficace d'au moins un composé de formule générale

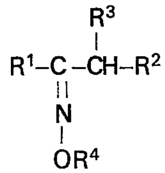

I

où $R^1$ représente un 2-halo-, un 4-halo- ou un 2,4-dihalo-phényle,

$R^2$ représente un 3-pyridyle ou un 2-pyrazinyle,

$R^3$ représente un hydrogène, ou un alcoyle en $C_1$ à $C_4$ à chaîne droite et

$R^4$ représente un alcoyle en $C_1$ à $C_6$, un cycloalcoyle en $C_3$ à $C_6$, un alcényle en $C_3$ à $C_6$ ou un alcynyle en $C_3$ à $C_6$, ou d'un sel d'addition d'acide d'un tel composé, ainsi qu'un support inerte.

2. Agent fongicide selon la revendication 1, où $R^1$ représente un 4-chlorophényle ou un 2,4-dichlorophényle.

3. Agent fongicide selon l'une des revendications 1 ou 2, où $R^3$ représente un hydrogène.

4. Agent fongicide selon la revendication 1, caractérisé en ce qu'il contient une quantité efficace de 2',4'-dichloro-2- (3-pyridyl)-acétophénone-O-méthyloxime ainsi qu'un support inerte.

5. Agent fongicide selon la revendication 1, caractérisé en ce qu'il contient une quantité efficace d'un composé de formule générale

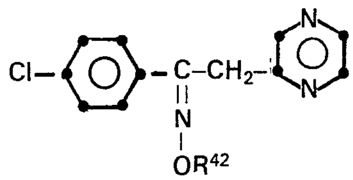

où $R^{42}$ représente un méthyle, un éthyle ou un allyle, ainsi qu'un support inerte.

6. Agent fongicide selon la revendication 1, caractérisé en ce qu'il contient une quantité efficace d'un composé de formule générale

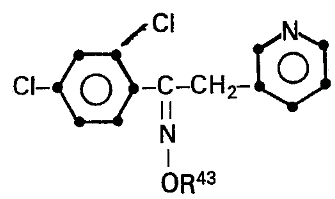

où $R^{43}$ représente un éthyle, un isopropyle ou un propargyle, ainsi qu'un support inerte.

7. Agent fongicide selon la revendication 1, caractérisé en ce qu'il contient une quantité efficace d'un composé de formule générale

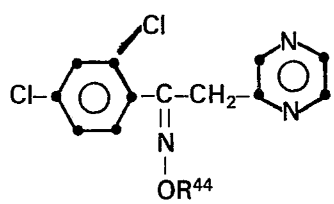

où $R^{44}$ représente un méthyle, un éthyle, un allyle ou un propargyle, ainsi qu'un support inerte.

8. Procédé de préparation de composés de formule générale

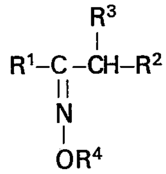

I

où

$R^1$ représente un 2-halo-, un 4-halo- ou un 2,4-dihalo-phényle,

$R^2$ représente un 3-pyridyle ou un 2-pyrazinyle,

$R^3$ représente un hydrogène ou un alcoyle en $C_1$ à $C_4$ à chaîne droite et

$R^4$ représente un alcoyle en $C_1$ à $C_6$, un cycloalcoyle en $C_3$ à $C_6$, un alcényle en $C_3$ à $C_6$ ou un alcynyle en $C_3$ à $C_6$, et de leurs sels d'addition d'acides, caractérisé en ce que

a) on fait réagir un oxime de formule

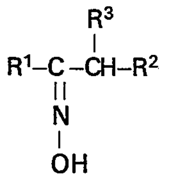

II

où $R^1$, $R^2$ et $R^3$ ont les significations données ci-dessus, avec un composé de formule

$R^4U$ III

où $R^4$ a la signification donnée ci-dessus et U représente un groupe sortant, ou

b) on fait réagir une cétone de formule

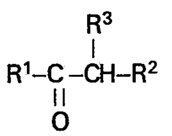

IV

où $R^1$, $R^2$ et $R^3$ ont les significations données ci-dessus, avec une hydroxylamine O-substituée de formule

$R^{41}ONH_2$ V

où $R^{41}$ représente un alcoyle en $C_1$ à $C_6$, un cycloalcoyle en $C_3$ à $C_6$ ou un alcényle en $C_3$ à $C_6$, et si on le désire en ce qu'on transforme un composé de formule I obtenu en un sel d'addition d'acide.

9. Procédé de préparation d'un agent fongicide, caractérisé en ce qu'on mélange au moins un des composés mentionnés dans les revendications 1 à 6 avec un support inerte.

10. Procédé de préparation d'un agent fongicide, caractérisé en ce qu'on mélange au moins un des composés mentionnés dans la revendication 7 avec un support inerte.

11. Procédé de lutte contre les champignons en agriculture et en horticulture, caractérisé en ce qu'on traite la matière à protéger avec une quantité efficace d'au moins un des composés mentionnés dans les revendications 1 à 6.

12. Procédé de lutte contre les champignons en agriculture et en horticulture, caractérisé en ce qu'on traite la matière à protéger avec une quantité efficace d'un des composés mentionnés dans la revendication 7.

13. Application d'un des composés ou agents mentionnés dans les revendications 1 à 6 à la lutte contre les champignons en agriculture et en horticulture.

14. Application d'un composé ou agent mentionné dans la revendication 7 à lutte contre les champignons en agriculture et en horticulture.

**Claims for the Contracting States: BE, CH, DE, FR, IT, LI, NL**

1. Compounds of the general formula

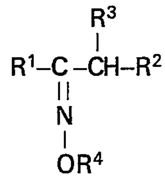

I

wherein $R^1$ signifies 2-halo-, 4-halo- or 2,4-dihalo-phenyl,

$R^2$ signifies 3-pyridyl or 2-pyrazinyl,

$R^3$ signifies hydrogen or straight-chain $C_{1-4}$-lkyl and

$R^4$ signifies $C_{1-6}$-alkyl, $C_{3-6}$-cycloalkyl, $C_{3-6}$-alkenyl or $C_{3-6}$-alkynyl, and acid addition salts of these compounds.

2. Compounds according to claim 1, wherein $R^1$ signifies 4-chlorophenyl or 2,4-dichlorophenyl.

3. Compounds according to claim 1 or 2, wherein $R^3$ signifies hydrogen.

4. 2',4'-Dichloro-2-(3-pyridyl)-acetophenone O-methyl oxime.

5. A compound according to claim 1, of the general formula

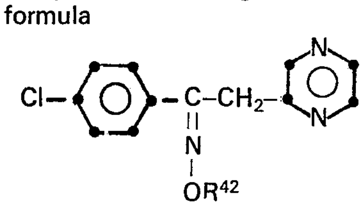

wherein $R^{42}$ signifies methyl, ethyl or allyl.

6. A compound according to claim 1, of the general formula

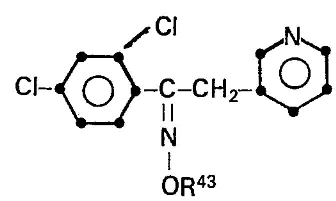

wherein $R^{43}$ signifies ethyl, isopropyl or propargyl.

7. A compound according to claim 1, of the general formula

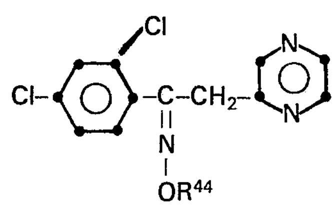

wherein $R^{44}$ signifies methyl, ethyl, allyl or propargyl.

8. Compounds according to any one of claims 1 to 7 as fungicidal active substances.

9. A fungicidal composition, characterized in that it contains an effective amount of at least one compound of general formula I according to claim 1 or an acid addition salt of such a compound as well as inert carrier material.

10. A fungicidal composition according to claim 9, characterized in that it contains an effective amount of 2',4'-dichloro-2-(3-pyridyl)-acetophenone-O-methyl oxime as well as inert carrier material.

11. A process for the manufacture of compounds of general formula I according to claim 1 and of their acid addition salts, characterized by

a) reacting on oxime of the formula

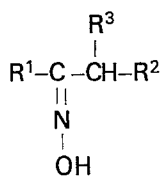 II

wherein $R^1$, $R^2$ and $R^3$ have the significances given above, with a compound of the formula

$R^4U$ III

wherein $R^4$ has the significance given above and U signifies a leaving group, or

b) reacting a ketone of the formula

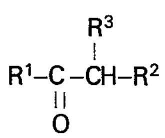 IV

wherein $R^1$, $R^2$ and $R^3$ have the significances given above, with a O-substituted hydroxylamine of the formula

$R^{41}ONH_2$ V

wherein $R^{41}$ signifies $C_{1-6}$-alkyl, $C_{3-6}$-cycloalkyl or $C_{3-6}$-alkenyl, and, if desired, converting a compound of formula I obtained into an acid addition salt.

12. A method for the control of fungi in agriculture and in horticulture, characterized by treating the locus to be protected with an effective amount of at least one of the compounds set forth in claims 1 to 7 or of one of the compositions set forth in claims 9 and 10.

13. The use of one of the compounds set forth in claims 1 to 7 or of one of the compositions set forth in claims 9 and 10 for the control of fungi in agriculture and in horticulture.

## Claims for the Contracting State: AT

1. A fungicidal composotion, characterized in that it contains an effective amount of at least one compound of the general formula

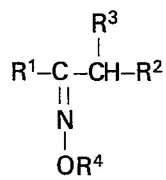 I

wherein

$R^1$ signifies 2-halo-, 4-halo- or 2,4-dihalophenyl,

$R^2$ signifies 3-pyridyl or 2-pyrazinyl,

$R^3$ signifies hydrogen or straight-chain $C_{1-4}$-alkyl and

$R^4$ signifies $C_{1-6}$-alkyl, $C_{3-6}$-cycloalkyl, $C_{3-6}$-alkenyl or $C_{3-6}$-alkynyl, or an acid addition salt of such a compound, as well as inert carrier material.

2. A fungicidal composition according to claim 1, wherein $R^1$ signifies 4-chlorophenyl or 2,4-dichlorophenyl.

3. A fungicidal composition according to claim 1 or 2, wherein $R^3$ signifies hydrogen.

4. A fungicidal composition according to claim 1, characterized in that it contains an effective amount of 2',4'-dichloro-2-(3-pyridyl)-acetophenone O-methyl oxime as well as inert carrier material.

5. A fungicidal composition according to claim 1, characterized in that it contains an effective amount of a compound of the general formula

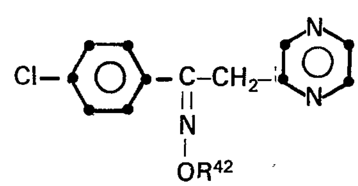

wherein $R^{42}$ signifies methyl, ethyl or allyl, as well as inert carrier material.

6. A fungicidal composition according to claim 1, characterized in that it contains an effective amount of a compound of the general formula

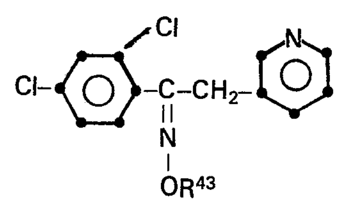

wherein $R^{43}$ signifies ethyl, isopropyl or propargyl, as well as inert carrier material.

7. A fungicidal composition according to claim 1, characterized in that it contains an effective amount of a compound of the general formula

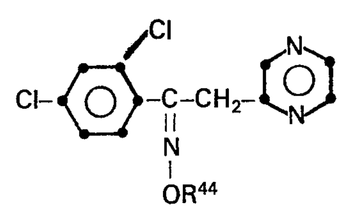

wherein $R^{44}$ signifies methyl, ethyl, allyl or propargyl, as well as inert carrier material.

8. A process for the manufacture of compounds of the general formula

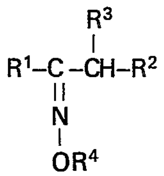

I

wherein

$R^1$ signifies 2-halo-, 4-halo- or 2,4-dihalophenyl,

$R^2$ signifies 3-pyridyl or 2-pyrazinyl,

$R^3$ signifies hydrogen or straight-chain $C_{1-4}$-alkyl and

$R^4$ signifies $C_{1-6}$-alkyl, $C_{3-6}$-cycloalkyl, $C_{3-6}$-alkenyl or $C_{3-6}$-alkynyl, and of their acid addition salts, characterized by

a) reacting an oxime of the formula

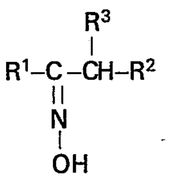

II

wherein $R^1$, $R^2$ and $R^3$ have the significances given above, with a compound of the formula

$$R^4U \qquad III$$

wherein $R^4$ has the significance given above and U signifies a leaving group, or

b) reacting a ketone of the formula

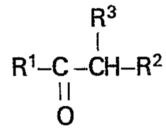

IV

wherein $R^1$, $R^2$ and $R^3$ have the significances given above, with a O-substituted hydroxylamine of the formula

$$R^{41}ONH_2 \qquad V$$

wherein $R^{41}$ signifies $C_{1-6}$-alkyl, $C_{3-6}$-cycloalkyl or $C_{3-6}$-alkenyl, and, if desired, converting a compound of formula I obtained into an acid addition salt.

9. A process for the manufacture of a fungicidal composition, characterized by mixing at least one of the compounds set forth in claims 1 to 6 with inert carrier material.

10. A process for the manufacture of a fungicidal composition, characterized by mixing at least one of the compounds set forth in claim 7 with inert carrier material.

11. A method for the control of fungi in agriculture and in horticulture, characterized by treating the locus to be protected with an effective amount of at least one of the compounds set forth in claims 1 to 6.

12. A method for the control of fungi in agriculture and in horticulture, characterized by treating the locus to be protected with an effective amount one of the compounds set forth in claim 7.

13. The use of one of the compounds or compositions set forth in claims 1 to 6 for the control of fungi in agriculture and in horticulture.

14. The use of one of the compounds or compositions set forth in claim 7 for the control of fungi in agriculture and in horticulture.